# EUROPEAN PATENT APPLICATION

(11) **EP 4 439 567 A1**
(43) Date of publication of application: **02.10.2024**
(21) Application number: 22897628.8
(22) Date of filing: 11.11.2022
(51) Int. Cl.: G16B 40/00

(54) **METHOD AND SYSTEM FOR PREDICTING EMBRYO TRANSPLANTATION RESULT OF ARTIFICIAL REPRODUCTION BY USING ARTIFICIAL INTELLIGENCE**

(30) Priority: 23.11.2021 US 202163282545 P
(71) Applicant: Sun, Sunny, Tainan City, Taiwan 704 (CN)
(72) Inventor: Sun, Sunny, Tainan City, Taiwan 704 (CN)
(74) Representative: Cabinet Chaillot
(86) International application number: PCT/CN2022/131317
(87) International publication number: WO 2023/093545

(57) **Abstract**

A system for predicting an implantation outcome of an embryo during in vitro fertilization treatment by using artificial intelligence includes an input interface and an artificial intelligence prediction model. A method for predicting the implantation outcome of an embryo during in vitro fertilization treatment by using artificial intelligence includes providing the input interface for inputting parameters, wherein the parameters include a genetic information of the embryo and a basic information of a woman who is going to be implanted with the embryo. The genetic information of the embryo includes a copy number of mitochondrial DNA and a telomere length. The basic information of the woman includes an age, an information of chromosome abnormality, and history of recurrent miscarriage. The artificial intelligence prediction model is provided to receive the parameters to generate a prediction result that is related to the implantation outcome.

## Description

### BACKGROUND OF THE INVENTION

### Technical Field

The present invention relates generally to predict embryos' implantation outcomes following embryo transfer in an in vitro fertilization (IVF) center, and more particularly to a method and a system for the prediction of implantation outcomes of an embryo during in vitro fertilization treatment using artificial intelligence.

### Description of Related Art

The low fertility rate is a significant public health issue around the world. In Taiwan, the female total fertility rate (namely, the life-time average number of children born per woman) fell from 7.04 in 1951 to 1.125 in 2016. Within a decade (from 2006 to 2016), the mean age of women with reproductive age rose from 25-29 years old (2006) to 30-34 years old (2016). Moreover, among women with reproductive age, the percentage of advanced maternal age increased from 11.84% in 2006 to 27.17% in 2017. Late marriage and advanced maternal age (≥34 years old) are the major reasons for low fertility and female infertility.

Nowadays, infertility is a common disease affecting up to 10% of all couples globally. In vitro fertilization (IVF) treatment has become one of the procedures to solve infertility problems. Although an IVF technique is well-developed, the advanced infertility group even receives in vitro fertilization (IVF) treatment only has about 30% successful rate, which is relatively low. Therefore, how to improve the success rate of the infertility group receiving IVF treatment is an urgent issue.

### BRIEF SUMMARY OF THE INVENTION

In view of the above, the primary objective of the present invention is to provide a method and a system to predict embryos' implantation outcomes following embryo transfer in an IVF center for predicting an implantation outcome of in vitro fertilization treatment by using artificial intelligence, in other words, which could predict the outcome of embryo implantation.

The present inventive subject matter provides a method to predict embryos' implantation outcomes following embryo transfer during IVF treatment using artificial intelligence, including the following steps:

An input interface is provided, wherein a plurality of parameters is entered through the input interface. The plurality of parameters includes a genetic information of an embryo and a basic information of a woman who is going to be implanted with the embryo. The genetic information of the embryo includes a copy number of mitochondrial DNA and a telomere length. The basic information of the woman includes an age, an information of chromosome abnormality, and a history of recurrent miscarriage.

An artificial intelligence prediction model is provided, wherein the artificial intelligence prediction model receives the plurality of parameters to generate a prediction result based on the plurality of parameters. The prediction result is related to the implantation outcome of the embryo.

The present inventive subject matter provides a system to predict embryos' implantation outcomes following embryo transfer during IVF treatment by using artificial intelligence, including an input interface and an artificial intelligence prediction model. The input interface is adapted to input a plurality of parameters, wherein the parameters includes a genetic information of an embryo and a basic information of a woman who is going to be implanted with the embryo. The genetic information of the embryo includes a copy number of mitochondrial DNA and a telomere length. The basic information of the woman includes an age, an information of chromosome abnormality, and a history of recurrent miscarriage. The artificial intelligence prediction model is adapted to receive the parameters to generate a prediction result based on the plurality of parameters, wherein the prediction result is related to an implantation outcome of the embryo.

With such design, the prediction result, which is obtained by using the method and the system of the present invention, could be taken into account as an auxiliary factor for determining whether or not to undergo the embryo implantation, thereby improving the success rate (pregnancy rate) of an embryo during in vitro fertilization treatment.

### BRIEF DESCRIPTION OF THE SEVERAL VIEWS OF THE DRAWINGS

The present invention will be best understood by referring to the following detailed description of some illustrative embodiments in conjunction with the accompanying drawings, in which
FIG. 1 is a flow chart of the method to predict embryos' implantation outcomes following embryo transfer in IVF treatment by using artificial intelligence of a first embodiment according to the present invention;
FIG. 2 is a block diagram of the system to predict embryos' implantation outcomes following embryo transfer in IVF treatment by using artificial intelligence of the first embodiment according to the present invention, showing the system is connected to the database; and
FIG. 3 is a ROC curve chart, showing the ROC curve for logistic regression and the ROC curve for decision tree models.

### DETAILED DESCRIPTION OF THE INVENTION

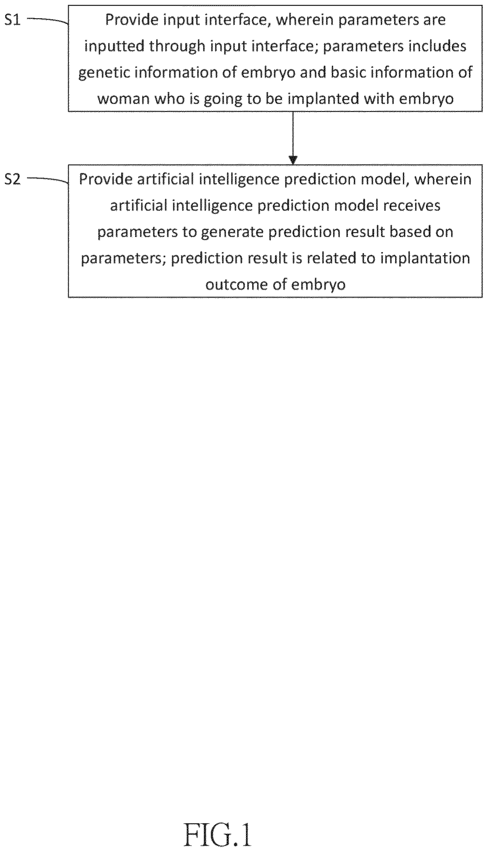

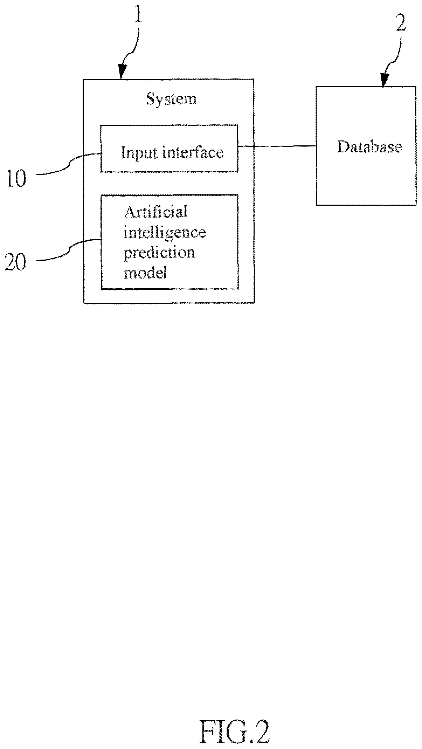

FIG. 1 shows a flowchart of a method for predicting an implantation outcome following embryo transfer during in vitro fertilization treatment by using artificial intelligence of a first embodiment according to the present invention, wherein the method is applied to system 1 shown in FIG. 2. The system 1 includes an input interface 10 and an artificial intelligence prediction model 20, and the input interface 10 is adapted to be connected to a database 2. The following describes the method and the system of the first embodiment at the same time.

Step S1: The input interface 10 is provided for entering a plurality of parameters, wherein the parameters include a genetic information of an embryo and a basic information of a woman who will be implanted with the embryo. In the current application, the embryo is a fertilized egg that is cultured to achieve a condition that is ready for implantation. In the current embodiment, a mother of the embryo (a provider of an egg for forming the embryo) and the woman who is implanted with the embryo are the same, but the mother and the woman are not limited to being the same. In other embodiments, the mother of the embryo and the woman who is implanted with the embryo could be different people.

In the current application, the genetic information of the embryo that needs to be evaluated and the basic information of the woman who will be implanted with the embryo could be stored in the database 2, so that the input interface retrieves or receives the parameters from the database 2. In an embodiment, the genetic information of the embryo and the basic information of the woman could be entered via the input interface.

Step S2: The artificial intelligence prediction model 20 is provided to receive the parameters through the input interface 10, wherein the artificial intelligence prediction model 20 generates a prediction result based on the parameters that are received, and the prediction result is related to an implantation outcome of the embryo.

The artificial intelligence prediction model 20 could be selected from a plurality of the artificial intelligence prediction models that are trained and predetermined. In the current embodiment, the artificial intelligence prediction model 20 is one of the predetermined artificial intelligence prediction models, which has the best predictive power. The prediction result could be a predicted implantation rate and/or a predicted pregnancy rate after the transfer of the embryo to the woman as an example. However, the prediction result is not limited to the predicted implantation rate and the predicted pregnancy rate, wherein the prediction result related to the implantation outcome of the embryo could further include a predicted implantation outcome (namely, successful implantation or non-successful implantation), a confidence level to the predicted implantation outcome, or a combination thereof.

In the current embodiment, the genetic information of the embryo includes the copy number of mitochondrial DNA (CN of mtDNA) and the telomere length (TL). The genetic information of the embryo is obtained by conducting genome sequencing to the embryo via Next Generation Sequencing (NGS) to obtain an sequencing result of embryo genome. Sequencing could be performed on any NGS platform, such as ThermoFisher platform or/and Illumina platform.

The copy number of mtDNA and the telomere length are calculated based on the sequencing result of embryo genome, so that a sophisticated process of quantification PCR (qPCR) for obtaining the telomere length could be avoided. For example, by using known repeated sequences of the telomere, such as TTAGGG or CCCTAA, to align with the sequencing result of embryo genome, an average telomere length of the sequencing result of embryo genome could be estimated. However, the means for obtaining the copy number of mtDNA and the telomere length is not limited to NGS, a person who is familiar to the field of genomics could choose a proper approach to obtain the copy number of mtDNA and the telomere length of the embryo.

The basic information of the woman, who is going to be implanted with the embryo, includes an age, an information of chromosome abnormality (CA), and a history of recurrent miscarriage (RM). The information of chromosome abnormality of the woman could be obtained via NGS, karyotyping, and array CGH. The information of chromosome abnormality could include information of chromosome abnormality of the woman and/or her family. The history of recurrent miscarriage could include history of recurrent miscarriage of the woman and/or her family.

In the current embodiment, the database 2 further includes a plurality of historical data, wherein each of the historical data includes a genetic information of a prioritized embryo and a basic information of a woman who is implanted the prioritized embryo. In the current embodiment, a mother of the prioritized embryo and the woman who is implanted the prioritized embryo are the same person. The genetic information of the prioritized embryo of each of the historical data includes a copy number of mtDNA and a telomere length, and the copy number of mtDNA and the telomere length are calculated based on a sequencing result of the prioritized embryo that is obtained via NGS. The basic information of the woman of each of the historical data includes age, information of chromosome abnormality of the woman and her family, and history of recurrent miscarriage of the woman and her family. The information of chromosome abnormality could be obtained via NGS, karyotyping, and array CGH. Each of the historical data includes an implantation outcome, wherein the implantation outcome shows either successful implantation or non-successful implantation.

The reason for choosing the copy number of mtDNA and the telomere length as the genetic information of the prioritized embryo is that when the information of chromosome abnormality of the woman who is implanted the prioritized embryo is aneuploid, a copy number of mtDNA of the prioritized embryo is higher than a copy number of mtDNA of an embryo having a euploid chromosome. A telomere length of an embryo that is successfully implanted is longer than a telomere length of an embryo that is non-successfully implanted. Since the copy number of mtDNA and the telomere length are significantly correlated to the implantation rate, the copy number of mtDNA and the telomere length are at least selected as the genetic information for training the predetermined artificial intelligence prediction models. Age and history of recurrent miscarriage of the woman who is implanted are factors that affect the implantation rate. Therefore, the age, the history of recurrent miscarriage, and the information of chromosome abnormality of the woman could be selected as the basic information for training the predetermined artificial intelligence prediction models. In the current embodiment, the age of the woman who is implanted with the prioritized embryo is in a range of 24 to 50 years old. A number of the prioritized embryos is 216, and each of the prioritized embryos is implanted to a corresponding one of the women, wherein the mother of each of the prioritized embryos is the woman who is implanted with that prioritized embryo.

In the current embodiment, the predetermined artificial intelligence prediction models could be trained by using algorithms of logistic regression, decision tree, and random forest as an example to explain, but the algorithm is not limited to the abovementioned three algorithms. In other embodiments, the algorithm could be selected at least one of the following algorithms, including support vector machine (SVM), XGBoots, artificial neural network (ANN), Bayesian network, Tree-based feature selection, deep learning, and so on.

Before the step S1 of the method of the current embodiment, a step of training the prediction models and a step of choosing one of the prediction models are included.

The step of training the prediction models includes taking a part of the historical data in the database 2 as a training data set to train each of the predetermined artificial intelligence prediction models. Anotheor part of the historical data in the database 2 is taken as a validation data set. In other words, each training information of the training data set includes a genetic information of the prioritized embryo and a basic information of the woman corresponding to the prioritized embryo.

When each of the predetermined artificial intelligence prediction models is trained, K fold cross validation could be used. In the current embodiment, the K value is equal to 5 (K=5), but it is not limited to 5.

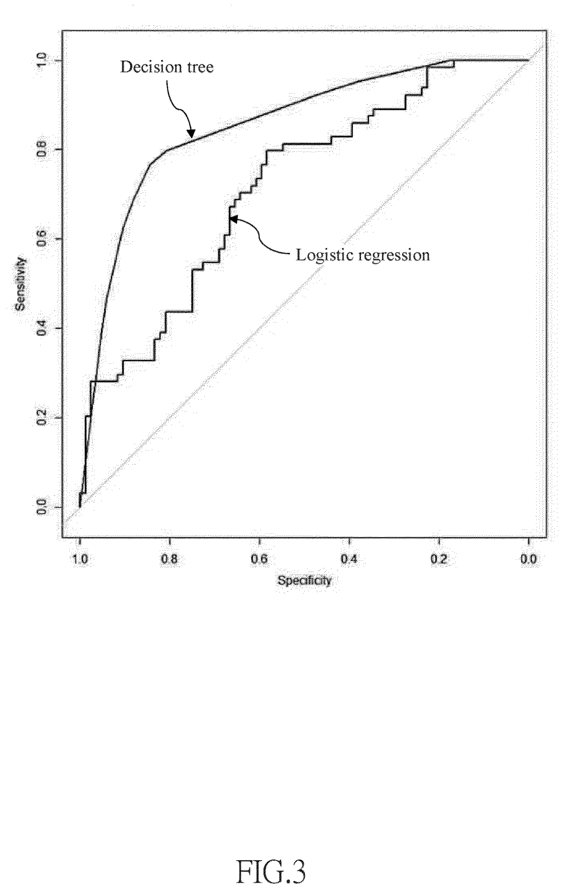

FIG. 3 shows a ROC curve of logistic regression and a ROC curve of decision tree. An accuracy of the artificial intelligence prediction model that is trained by logistic regression is 0.65 (65%), and an area under ROC curve (AUC) thereof is 0.72 (72%). An accuracy of the artificial intelligence prediction model that is trained by the decision tree is 0.81 (81%), and an area under ROC curve thereof is 0.86 (86%).

As to the artificial intelligence prediction model that is trained by random forest, wherein the random forest classification is based on 500 decision trees as an example for explanation. An accuracy of the artificial intelligence prediction model that is trained by random forest is 0.91 (91%), which is higher than either 0.81 (81%) of the accuracy of the artificial intelligence prediction model that is trained by the decision tree (a single decision tress) or 0.65 (65%) of the accuracy of the artificial intelligence prediction model that is trained by logistic regression. In other words, the predictive ability of the artificial intelligence prediction model that is trained by random forest is preferred. By using the validation data set to test a performance of each of the artificial intelligence prediction models, wherein the performance of the artificial intelligence prediction model trained by random forest is 0.61 (61%), and the performance of the artificial intelligence prediction model trained by decision tree is 0.54 (54%).

The step of choosing one of the prediction models includes choosing one of the predetermined artificial intelligence prediction models, which has a preferred predictive ability to be the artificial intelligence prediction model 20. According to the description above, the predictive ability of the artificial intelligence prediction model trained by random forest is preferred, so that the artificial intelligence prediction model trained by random forest is chosen to be the artificial intelligence prediction model 20 of the system for predicting implantation outcome of IVF treatment. In other words, the artificial intelligence prediction model 20 is trained by using the training data set.

After the artificial intelligence prediction model 20 receives the parameters through the input interface 10, the prediction result could be generated. A medical personnel could refer to the prediction result generated by the artificial intelligence prediction model 20 to determine whether or not to implant the embryo in the woman.

A method and a system for predicting the implantation outcome following embryo transfer during in vitro fertilization treatment by using artificial intelligence of a second embodiment according to the present invention is described below. The method and the system of the second embodiment are almost the same as that of the first embodiment, except for the parameters that are utilized for predicting the implantation outcome. Except for the genetic information of the embryo (CN of mtDNA and telomere length) and the basic information of the woman (age, information of chromosome abnormality, and history of recurrent miscarriage) in the first embodiment, the parameters of the second embodiment further includes the following parameters:

The genetic information of the embryo further includes a chromosome stability, wherein the chromosome stability is a value that indicates an overall dispersion of the sequencing result of NGS data.

The basic information of the woman further includes a family disease history, a cause of infertility, and a history of gynecological disease. The family disease history includes abortion history, immune abnormality, ectopic pregnancy, preterm birth, prematurity, and a combination thereof. The cause of infertility includes a factor caused by fallopian tube abnormalities, a factor caused by ovulation disorders, a factor caused by uterine or cervical abnormalities, causes of male infertility, and a combination thereof. However, the causes of infertility are not limited to the above-mentioned. The history of gynecological disease includes endometriosis, polycystic ovary syndrome (PCO), cervical myoma, endometrial polyps, adenomyosis, ovarian cyst, oviduct obstruction, other cervical or fallopian tube abnormalities, and a combination thereof. The basic information of the woman could further include a history of gynecological examination and surgery of the woman, wherein the history of gynecological examination and surgery of the woman includes resectoscope, laparoscopy, dilatation and curettage, and a combination thereof. However, the history of gynecological examination and surgery of the woman is not limited to the above-mentioned.

The parameters further include an information about physical characteristics of the woman and a treatment record. The information about physical characteristics of the woman includes endometrial thickness and menstrual cycle. The treatment record includes an egg source (from the woman herself or be donated by another woman), a blastomere size and/or grade, a sum of a number of fertilized eggs and an number of fertilized eggs implanted. In an embodiment, when there is merely one fertilized egg is formed and implanted, the sum of the number of fertilized eggs and the number of fertilized eggs implanted is one.

A basic information of a father of the embryo, namely a provider of sperm, includes the information of chromosome abnormality, wherein the information of chromosome abnormality could be an information of chromosome abnormality of the father and/or his family.

In an embodiment, the physical characteristics of the woman could further include a hormone level, wherein the hormone could include AMH, FSH, LH, E2, TSH, P4, PRL, and a combination thereof. The treatment record could further include an ovarian stimulation method, a number of eggs collected from the woman, a number of fertilized eggs of the woman, a morphology of the embryo, and a morphokinetics of the embryo. Optionally, the basic information of the father could further include a history of recurrent miscarriage of the father's family.

Each of the historical data in the database 2 not only includes the genetic information of the prioritized embryo (CN of mtDNA and telomere length), the basic information of the woman who is implanted with the prioritized embryo (age, information of chromosome abnormality, and history of recurrent miscarriage), and the implantation outcome, but also includes the information about physical characteristics of the woman, the treatment record of the woman, and the basic information of the father of the embryo. The parameters used in the second embodiment are described as above, so that we are not going to describe in details herein. In the current embodiment, a number of the historical data is 456, wherein the historical data is related to 456 embryos that are obtained from 239 women.

In the current embodiment, the genetic information of the embryo is obtained by conducting genome sequencing to the embryo via Next Generation Sequencing (NGS) to obtain an sequencing result of embryo genome. Sequencing could be performed on ThermoFisher platform and Illumina platform, so that two sets of the genetic information of the embryo could be obtained based on the sequencing result acquired through the ThermoFisher platform and the sequencing result acquired through the Illumina platform.

In the current embodiment, the predetermined artificial intelligence prediction models could be trained by using one of the algorithms, including logistic regression, decision tree, random forest support vector machine (SVM), LightGBM, XGBoots, Tabnet, and ensemble learning. When the predetermined artificial intelligence prediction model trained by the ensemble learning, the predetermined artificial intelligence prediction model is simultaneously trained by the 7 algorithms mentioned above. However, the algorithms are not limited to the abovementioned, other algorithms could be utilized or ensembled.

Each of the historical data in the database 2 includes the implantation outcome, namely whether or not the woman who is implanted become pregnant. The implantation outcome is determined by three measurements, including a hormone blood test, an ultrasonic inspection, and a result of whether born a baby or not.

In the current embodiment, the step of training the prediction models includes taking a part of the historical data in the database 2 as a training data set to train each of the predetermined artificial intelligence prediction models. Anotheor part of the historical data in the database 2 is taken as a validation data set. When each of the predetermined artificial intelligence prediction models is trained, K fold cross validation could be used. In the current embodiment, the K value is equal to 3 (K=3), but it is not limited to 3.

More specifically, each of the predetermined artificial intelligence prediction models is trained by 9 training data sets and validated by a validation data set. The first to third training data sets and the validation data set include the genetic information of the prioritized embryos that are obtained by using the Thermo Fisher platform and the implantation outcomes of the prioritized embryos that are determined by the three methods. The fourth to sixth training data sets and the validation data set include the genetic information of the prioritized embryos that are obtained by using the Illumina platform and the implantation outcomes of the prioritized embryos that are determined by the three methods. The seventh to ninth training data sets and the validation data set include the genetic information of the prioritized embryos that are obtained by simultaneously using the Thermo Fisher platform and the Illumina platform and the implantation outcomes of the prioritized embryos that are determined by the three methods.

The artificial intelligence prediction model 20 of the current embodiment is selected from 72 predetermined artificial intelligence prediction models that are trained, wherein the selected one of the artificial intelligence prediction model 20 has a preferred predictive ability. The 72 predetermined artificial intelligence prediction models are obtained by using 9 training data sets to train 8 predetermined artificial intelligence prediction models.

For example, each of the training data sets is used for training 8 predetermined artificial intelligence prediction models, and then one of the artificial intelligence prediction models that has the preferred predictive ability is selected. Since there are 9 training data sets, the 9 predetermined artificial intelligence prediction models are preliminarily selected. Next, one of the 9 predetermined artificial intelligence prediction models that has the best predictive power is selected to be the artificial intelligence prediction model 20.

The selected one of the 72 predetermined artificial intelligence prediction models is the predetermined artificial intelligence prediction model that is trained by ensemble learning, wherein the training data sets used for training include the genetic information of the prioritized embryos obtained through Thermo Fisher platform and the implantation outcomes of the prioritized embryos.

In the second embodiment, when the training data sets are used to test the performance of the artificial intelligence prediction model 20 that is finally selected, an accuracy of the artificial intelligence prediction model 20 is 97.6%, a sensitivity of the artificial intelligence prediction model 20 is 95.0%, a positive predictive value (PPV) is 97.4%, a F1 value is 96.2%, and an area under ROC curve of the artificial intelligence prediction model 20 is 99.6%. When the validation data set is used to test the performance of the artificial intelligence prediction model 20, an accuracy of the artificial intelligence prediction model 20 is 87.5%, a sensitivity of the artificial intelligence prediction model 20 is 78.3%, a positive predictive value (PPV) is 82.5%, a F1 value is 80.3%, and an area under ROC curve of the artificial intelligence prediction model 20 is 87.5%.

The artificial intelligence prediction model 20, which is trained by ensemble learning, has the best predictive power for predicting the implantation outcome of IVF treatment, which could be referred to to evaluate whether each of the embryos is suitable for implantation or not, thereby increasing the success rate of IVF treatment.

It must be pointed out that the embodiment described above is only a preferred embodiment of the present invention. All equivalent structures and methods which employ the concepts disclosed in this specification and the appended claims should fall within the scope of the present invention.

## Claims

1. A method for predicting an implantation outcome of an embryo during in vitro fertilization treatment by using artificial intelligence, comprising the following steps:
providing an input interface, wherein a plurality of parameters is entered through the input interface, the plurality of parameters comprises a genetic information of an embryo and a basic information of a woman who is going to be implanted with the embryo; the genetic information of the embryo comprises a copy number of mitochondrial DNA and a telomere length; the basic information of the woman comprises an age, an information of chromosome abnormality, and a history of recurrent miscarriage; and
providing an artificial intelligence prediction model, wherein the artificial intelligence prediction model receives the plurality of parameters to generate a prediction result based on the plurality of parameters; the prediction result is related to the implantation outcome of the embryo.

2. The method as claimed in claim 1, wherein the plurality of parameters comprises an information of chromosome abnormality of a father of the embryo.

3. The method as claimed in claim 1, comprising sequencing the embryo via next generation sequencing (NGS) to obtain the genetic information of the embryo.

4. The method as claimed in claim 3, wherein the telomere length and the copy number of mitochondrial DNA of the genetic information of the embryo is calculated based on a sequencing result obtained via NGS.

5. The method as claimed in claim 1, wherein the genetic information of the embryo comprises a chromosome stability.

6. The method as claimed in claim 1, wherein the basic information of the woman comprises a family disease history, a cause of infertility, and a history of gynecological disease.

7. The method as claimed in claim 6, wherein the basic information of the woman comprises a history of examination and surgery.

8. The method as claimed in claim 1, wherein the plurality of parameters further comprises an information about physical characteristics of the woman, and the information about physical characteristics of the woman comprises an endometrial thickness.

9. The method as claimed in claim 8, wherein the information about physical characteristics of the woman comprises a hormone level.

10. The method as claimed in claim 8, wherein the information about physical characteristics of the woman comprises a menstrual cycle.

11. The method as claimed in claim 1, wherein the plurality of parameters further comprises a treatment record, and the treatment record comprises an egg source, a blastomere grade, and a sum of a number of fertilized eggs implanted and a number of fertilized eggs.

12. The method as claimed in claim 11, wherein the treatment record comprises an ovarian stimulation method, a number of eggs collected from the woman, a number of fertilized eggs of the woman, a morphology of the embryo, and a morphokinetics of the embryo.

13. The method as claimed in claim 1, further comprising training the artificial intelligence prediction model by using a training data set, wherein the training data set comprises a plurality of training information; each of the plurality of training information comprises a genetic information of a prioritized embryo and a basic information of a woman corresponding to the prioritized embryo; the genetic information of the prioritized embryo comprises a copy number of mtDNA and a telomere length; the basic information of the woman corresponding to the prioritized embryo comprises an age, an information of chromosome abnormality, history of recurrent miscarriage.

14. The method as claimed in claim 1, wherein the artificial intelligence prediction model is trained by using one of a plurality of algorithms comprising logistic regression, decision tree, random forest, support vector machine (SVM), LightGBM, XGBoots, Tabnet, and ensemble learning.

15. A system for predicting implantation outcome of an embryo during in vitro fertilization treatment by using artificial intelligence, comprising
an input interface adapted to input a plurality of parameters, wherein the plurality of parameters comprises a genetic information of an embryo and a basic information of a woman who is going to be implanted with the embryo; the genetic information of the embryo comprises a copy number of mitochondrial DNA and a telomere length; the basic information of the woman comprises an age, an information of chromosome abnormality, history of recurrent miscarriage;
an artificial intelligence prediction model adapted to receive the plurality of parameters to generate a prediction result based on the plurality of parameters, wherein the prediction result is related to an implantation outcome of the embryo.

16. The system as claimed in claim 15, wherein the plurality of parameters comprises an information of chromosome abnormality of a father of the embryo.

17. The system as claimed in claim 16, wherein the basic information of the woman comprises a family disease history, a cause of infertility, and a history of gynecological disease.

18. The system as claimed in claim 15, wherein the plurality of parameters further comprises an information about physical characteristics of the woman, and the information about physical characteristics of the woman comprises an endometrial thickness.

19. The system as claimed in claim 15, wherein the plurality of parameters further comprises a treatment record, and the treatment record comprises an egg source, a blastomere grade, and a sum of a number of fertilized eggs implanted and a number of fertilized eggs.

20. The system as claimed in claim 15, wherein the genetic information of the embryo further comprises a chromosome stability.
